# EUROPEAN PATENT APPLICATION

(11) **EP 3 054 389 A2**
(43) Date of publication of application: **10.08.2016**
(21) Application number: 16152412.9
(22) Date of filing: 22.01.2016
(51) Int. Cl.: G06F 19/00

(54) **WOUND MONITORING**

(30) Priority: 06.02.2015 US 201514615842
(71) Applicant: NXP B.V., 5656 AG Eindhoven (NL)
(72) Inventor: NACKAERTS, Axel, 5656 AG Eindhoven (NL); FREDERIX, Filip, 5656 AG Eindhoven (NL); DE TROCH, Stefan, 5656 AG Eindhvoen (NL); DAVID, Johan, 5656 AG Eindhoven (NL)
(74) Representative: Miles, John Richard

(57) **Abstract**

As may be implemented with one or more embodiments herein, aspects of the disclosure are directed to an apparatus having one or more sensor circuits and a wound-characterization circuit that generates data that characterizes a condition of the wound based on physiological characteristics detected by the sensors. A wireless communication circuit wirelessly transmits the characterization data, which can be carried out while the wound is covered. In various implementations, an integration circuit (e.g., connecting circuitry and/or a chip) includes the wound-characterization circuit and the sensor circuit, and further couples to a wound dressing for sensing the different physiological characteristics of the wound while the wound dressing covers a portion of the wound from which the different physiological characteristics are sensed.

## Description

Aspects of various embodiments are directed to wound monitoring, and to doing so using multiple electronic sensors.

For a variety of wounds ranging from simple cuts or scrapes to more serious wounds such as larger cuts or scrapes, burns and wounds resulting from medical procedures, monitoring the status of the wound can be important for proper healing and to address adverse conditions such as infection. Generally, monitoring wounds involves visual inspection, which can be carried out by a patient or medical professional. For more serious wounds in particular, monitoring by a medical professional can be important.

Wound monitoring can be challenging for a variety of reasons. For instance, inspecting wounds generally requires removal of a bandage or dressing-type component that is used to cover the wound. Further, while patient monitoring can be useful, it can often be desirable to have a medical practitioner monitor a wound to provide additional expertise. Unfortunately, monitoring by a medical practitioner can be expensive (e.g., requiring hospital stays or in-home visits), impractical or otherwise difficult for patients who may need to travel to visit a medical practitioner, and untimely. In addition, visual monitoring can be somewhat limited in assessing wound conditions. These and other matters have presented challenges to medical care, for a variety of applications.

Various example embodiments are directed to wound-monitoring circuits and their implementation.

According to an example embodiment, an apparatus includes a sensor circuit and a wound-characterization circuit that responds to signals provided from the sensor circuit, by generating characterization data that characterizes a condition of the wound based on physiological characteristics represented by the respective signals. The apparatus also includes a wireless communication circuit that operates with the wound-characterization circuit to wirelessly transmit the characterization data, and a power circuit that provides power to the wound-characterization circuit and to the wireless communication circuit. An integration circuit includes the wound-characterization circuit and the sensor circuit, and couples to a wound dressing for sensing the different physiological characteristics of the wound while the integration circuit and the wound dressing cover at least a portion of the wound.

Another example embodiment is directed to an apparatus including first and second circuits as follows. The first circuit couples to a wound dressing and includes a plurality of sensors, a wound-characterization circuit and a wireless communication circuit. The sensors senses different physiological characteristics of a wound, while the wound dressing covers at least a portion of the wound from which the physiological characteristics are sensed. The wound-characterization circuit responds to signals provided from the sensors by generating data that characterizes a condition of the wound based on physiological characteristics represented by the respective signals. The wireless communication circuit operates with the wound-characterization circuit to wirelessly transmit the characterization data to the second circuit, which receives the characterization data and provides an indication of a condition of the wound based thereupon. In some implementations, the second circuit powers the first circuit via wireless power that the first circuit harvests and uses to operate the sensors, wound-characterization circuit and wireless communication circuit.

In further implementations, the second circuit remotely controls the first circuit by transmitting operational instructions for controlling selected ones of the sensors for sensing specific physiological characteristics. The first circuit is responsive to the operational instructions by operating the sensors to sense the specific physiological characteristics and by generating characterization data, via the wound-characterization circuit, that characterizes a condition of the wound based on the operational instructions.

Another example embodiment is directed to a method as follows. Using a sensor circuit integrated with a wound dressing that covers portions of a wound, physiological characteristics of the covered portions of the wound are sensed and respective signals that characterize the physiological characteristics are provided. Using a wound-characterization circuit integrated with the sensor circuit and coupled to the wound dressing, characterization data that characterizes a condition of the wound is generated based on physiological characteristics represented by the respective signals. These aspects are carried out while the wound dressing covers the portions of the wound, which facilitates monitoring of the wound without necessarily removing a wound dressing. In certain implementations, two or more different physiological characteristics of the wound are sensed and the characterization data is generated based on a combination of the different physiological characteristics. Other implementations further include receiving and using the wirelessly transmitted characterization data to identify a condition of the wound, and generating an alert that alerts a user of adverse conditions while the wound dressing covers the portion of the wound, in response to the identified condition of the wound indicating such an adverse condition.

The above discussion/summary is not intended to describe each embodiment or every implementation of the present disclosure. The figures and detailed description that follow also exemplify various embodiments.

Various example embodiments may be more completely understood in consideration of the following detailed description in connection with the accompanying drawings, in which:
FIG. 1 shows an apparatus for wound monitoring, in accordance with an example embodiment;
FIG. 2 shows a circuit apparatus for wound monitoring, in accordance with another example embodiment; and
FIG. 3 shows a data flow diagram, in accordance with another example embodiment.

While various embodiments discussed herein are amenable to modifications and alternative forms, aspects thereof have been shown by way of example in the drawings and will be described in detail. It should be understood, however, that the intention is not to limit the invention to the particular embodiments described. On the contrary, the intention is to cover all modifications, equivalents, and alternatives falling within the scope of the disclosure including aspects defined in the claims. In addition, the term "example" as used throughout this application is only by way of illustration, and not limitation.

Aspects of the present disclosure are believed to be applicable to a variety of different types of apparatuses, systems and methods involving wound monitoring. While not necessarily so limited, various aspects may be appreciated through a discussion of examples using this context.

Various embodiments are directed to circuits and methods for wound care monitoring using electronic sensors. Certain embodiments employ a control circuit *(e.g.,* a microcontroller), a memory, a wireless interface (e.g., near-field communication (NFC)) and sensors that provide different sensor functionalities, all of which may be mounted on a common chip. Different ones of the sensors are used to detect different biological characteristics, which are used to provide an indication of a state or condition of the wound. These detected characteristics, or other data that provides an indication of the detected characteristics, is communicated via the wireless interface and thus provided for use in monitoring the wound.

The sensing circuitry and related circuits may be powered using one or more of a variety of approaches. In some implementations, battery power is used to operate the sensors, such as with printed batteries or coin cells. In other implementations, wireless power such as power provided with NFC signals is used, which can also be used to read out sensed characteristics.

Various sensors as described herein are implemented for sensing different biochemical characteristics of a wound, which may provide an indication of a complex series of biochemical events corresponding to wound healing. As examples, the sensors may detect or otherwise provide an indication of one or more of oxygen, moisture, glucose, lactate, color, conductivity, temperature and bacterial content. For instance, in some embodiments oxygen is detected using oxygen-sensitive materials, with a corresponding digital output provided for processing and/or communication. In certain embodiments, moisture and/or conductivity is measured, such as by using non-functionalized electrodes. Glucose, lactate or other parameters can be measured using an enzyme reaction and electrodes functionalized with enzymes that give an electrochemical reaction with the substrates of interest. Color can be measured with a photosensor and, in some implementations, involves generating light *(e.g.,* an LED or multiple LEDs) to illuminate the wound. Temperature sensors can provide an indication of wound healing status and inflammatory reactions, and can be implemented on a chip with a microcontroller and other circuits when the chip is in close proximity with the wound. In some implementations, bacterial content is detected optically using color-sensitive reactions and color sensing as noted above, or using a pH measurement (e.g., using a pH-sensitive material such as Ta₂O₅. One or more of these characteristics can be measured individually or in combination, with an output provided to characterize the wound. For general information regarding sensing wound characteristics, and for specific information regarding specific characteristics and combinations of characteristics that may be sensed to characterize a wound in accordance with one or more embodiments herein, reference may be made to Dargaville, T.R., et al., "Sensors and imaging for wound healing: A review," Biosensors and Bioelectronics (2012), http://dx.doi.org/10.1016/j.bios.2012.09.029, which is fully incorporated herein by reference.

In a particular embodiment involving local communications such as NFC communications, sensor circuity carrying out wound analysis or monitoring, operates with an NFC-compatible device such as an NFC-enabled smartphone, for providing wound characterization while the wound is covered with a wound care product. The sensor circuitry may be on or integrated in the wound care product, such as by printing on the wound care product via flexfoil or other printing techniques. These approaches facilitate wound monitoring by a medical practitioner or a patient at regular time intervals, using a smartphone or similar NFC-compatible device. Local monitoring in this regard may further be extended to greater distances, using an NFC-compatible device's long-range communication capabilities (e.g., cellular and/or Wi-Fi networks), using an application running on the NFC-compatible device. A status or other condition of the wound may be assessed in this regard, without necessarily removing some or all of a wound care product that covers the wound. An antenna and related circuitry that carries out sensing functions as characterized herein can be integrated into or onto the wound care product, and operable therewith to assess the wound while the wound care product is in place and hindering direct observation and monitoring of the wound.

In some embodiments, sensor data is stored in the memory of sensor circuitry as discussed herein. This data may be read out at a later time, or processed on the sensor circuitry (*e.g.,* via algorithms). In some implementations, wound characteristics as sensed over time and stored, are processed to evaluate time-based changes or other conditions of the wound. This information can be used to provide an indication of a condition of the wound, such as to provide an indication that a wound dressing should be removed for inspection or that other treatments should be applied.

In some embodiments, a local device having NFC or other local wireless communication capabilities is programmed to interact with sensor circuitry as characterized herein, and to control the sensor circuitry based on remote instructions received over a long-range network (*e.g.*, cellular and/or Wi-Fi networks). For instance, where a particular wound type or wound condition is to be monitored, a remote device may transmit control signals to the local device over a long-range network, and the local device in turn transmits operational commands or other instructions to the sensor circuitry via the local wireless connection. For example, simple wound monitoring may involve measurement of temperature only. A more advanced monitoring approach may measure moisture, conductivity and humidity. Even more advanced monitoring involves detecting bacterial content, glucose, lactate or oxygen. Such an approach may involve, for example, remotely analyzing wound characteristics obtained using the sensor circuitry and communicated via the local and long-range communications, and sending commands back to the sensor circuitry via the local device to modify the manner in which the sensor circuitry operates.

In certain embodiments as may be particularly applicable for severe wounds, sensor circuitry as characterized herein provides an alarm function that operates based on detected wound characteristics. Different physiological characteristics are monitored and used to assess a wound with regard to conditions that would warrant an alarm. For instance, where temperature and color of a wound combine to indicate an infection, an alarm such as a visible (e.g., thermochromic, electrochromic or LEDs) or audible communication, or a wireless communication indicative of the alarm, can be provided locally and/or remotely. Where implemented with a handheld device such as a mobile telephone, the alarm may involve using visual or audio characteristics of the mobile telephone. The sensor circuitry may be integrated in a wound care product for such an approach, and may include additional circuitry integrated with and/or separate from the sensor circuitry to carry out alarm functions.

In another embodiment, sensor circuitry as characterized herein operates to perform wound assessment at a regular interval, independent from external powering. For instance, such an approach may involve an internal battery that powers the sensor circuitry, for making the regular interval assessments. Such an approach may be implemented where monitoring at such intervals can be important, such as for severe or complex wounds.

A variety of form factors can be implemented for measuring certain parameters with sensor circuitry as discussed herein. In some embodiments, sensors are integrated in a chip for sensing characteristics such as temperature, humidity or color *(e.g.,* a photodiode). In these contexts, the chip may be directly in contact with the wound, or with a membrane or other material between the sensor and wound, and which allows fluid to diffuse towards the sensor and/or other access (e.g., passes light). In other embodiments, external sensors are connected to a sensor chip that processes and communicates information indicative of sensed characteristics. In such embodiments, sensors may be located at different portions of a wound, and can be located separate from the processing circuitry (e.g., with the processing circuitry integrated on an outer surface of a wound dressing, and sensors on an inner surface of the wound dressing that faces the wound).

In certain embodiments, sensor circuitry as characterized herein operates to sense light and/or pressure to provide an indication of positioning of the sensor relative to the wound. For instance, light measurements can indicate if a bandage is mechanically well-positioned and tight enough. Pressure sensors can be used to provide an indication of connectivity and/or placement of a bandage.

Various different sensing approaches may be used, as may various different types of wound dressings. For general information regarding sensing, and for specific information regarding sensing approaches that may be implemented in accordance with one or more embodiments herein, reference may be made to Dargaville, et al., "Sensors and imaging of wound healing" in Biosensors and Bioelectronics, Vol. 41, pp. 30-42 (March 15, 2013); reference may also be made to U.S. Pat. No. 7,625,117 (e.g., and the various bandages shown therein), both of which are fully incorporated herein by reference.

According to another example embodiment, a wound-characterization circuit responds to signals provided from one or more sensor circuits by generating characterization data that characterizes a condition of a wound based on physiological characteristics represented by the respective signals. The sensor circuits may, for example, include a plurality of sensors with each sensor operating to sense a physiological characteristic that is different than a physiological characteristic sensed by at least another one of the sensors. A wireless communication circuit wirelessly transmits the characterization data, and a power circuit provides power to the wound-characterization circuit and to the wireless communication circuit.

An integration circuit, such as an integrated circuit chip and/or a substrate, includes the wound-characterization circuit and the sensor circuit and couples to a wound dressing, and may affix the sensor circuit, wound-characterization circuit, wireless communication circuit and power circuit relative to one another. The different physiological characteristics of the wound can thus be sensed while the wound dressing covers at least a portion of the wound, and read out accordingly (e.g., without having to remove the wound dressing).

The wound-characterization circuit is implemented in a variety of manners. In some embodiments, the wound-characterization circuit generates the characterization data by converting the signals into digital data and executing an algorithm using the digital data as inputs, based upon the type of physiological characteristics represented by the respective signals. The resulting characterization data thus characterizes a condition of the wound based on a combination of different physiological characteristics represented by respective ones of the at least two signals. In certain implementations, the wound-characterization circuit processes data from the sensor circuit to determine a characteristic of the wound, and generates and transmits an alert via the wireless communication circuit in response to the determined characteristic of the wound indicating a medical condition defined as being adverse.

The sensors are implemented in a variety of manners, to suit particular embodiments. In some embodiments, each of a plurality of sensors characterize physiological characteristics that are different than a physiological characteristic sensed by another one of the sensors. The physiological characteristics may, for example, include two or more characteristics selected from the group of: an electrical characteristic, a chemical characteristic, a mechanical characteristic, a temperature characteristic and an optical characteristic. In certain implementations, two or more sensors are spaced apart from one another and operate with an integration circuit as above, to characterize different regions of the wound that are spaced apart from one another.

In a more particular embodiment, a memory circuit stores data representing wound condition-sensing protocols specific to different wound types, each protocol specifying a combination of sensors to be used. The wound-characterization circuit is a logic circuit that monitors different types of wounds by retrieving one of the sensing protocols specific to a particular type of wound, based upon identification data specifying the type of wound to be sensed, and operating different combinations of the plurality of sensors by retrieving and executing one of the protocols based on the identification data. In certain implementations, the logic circuit processes data from the sensors to determine a characteristic of the wound, and generates and transmits an alert via the wireless communication circuit in response to the determined characteristic of the wound indicating a medical condition defined in the memory as being adverse.

In another more particular embodiment, the wireless communication circuit is a NFC circuit that operates with the power circuit to receive wireless power, and uses the wireless power to power to the wound-characterization circuit and to the wireless communication circuit. The wound-characterization circuit operates the sensor circuit to obtain the physiological characteristics and to transmit the characterization data via the NFC circuit. Certain embodiments further employ a portable device having an NFC circuit and operating to transmit the wireless power to the wireless communication circuit via the NFC circuits, and remotely operate the sensor circuit and the wound-characterization circuit for detecting the physiological characteristics by communicating instructions to the wound-characterization circuit via the NFC circuits. In more particular embodiments, the portable device remotely operates the sensors, the NFC circuit and the wound-characterization circuit by controlling the wound-characterization circuit to operate at least a first one of the sensors for detecting a biochemical characteristic of the wound, and to transmit the characterization data to the portable device. In response to the characterization data, the portable device controls the wound-characterization circuit to operate a different one of the sensors for detecting a physiological characteristic of the wound, and transmit data including the sensed biochemical characteristic of the wound back to the portable device.

Certain embodiments further include a wound dressing that operates with an integration circuit as above, to support and adhere the sensor circuit, wound-characterization circuit, wireless communication circuit and power circuit in contact with a patient exhibiting the wound. The wound dressing, as augmented with this circuitry, provides assessment of a portion of the wound covered by the wound dressing while the wound dressing is covering the portion of the wound.

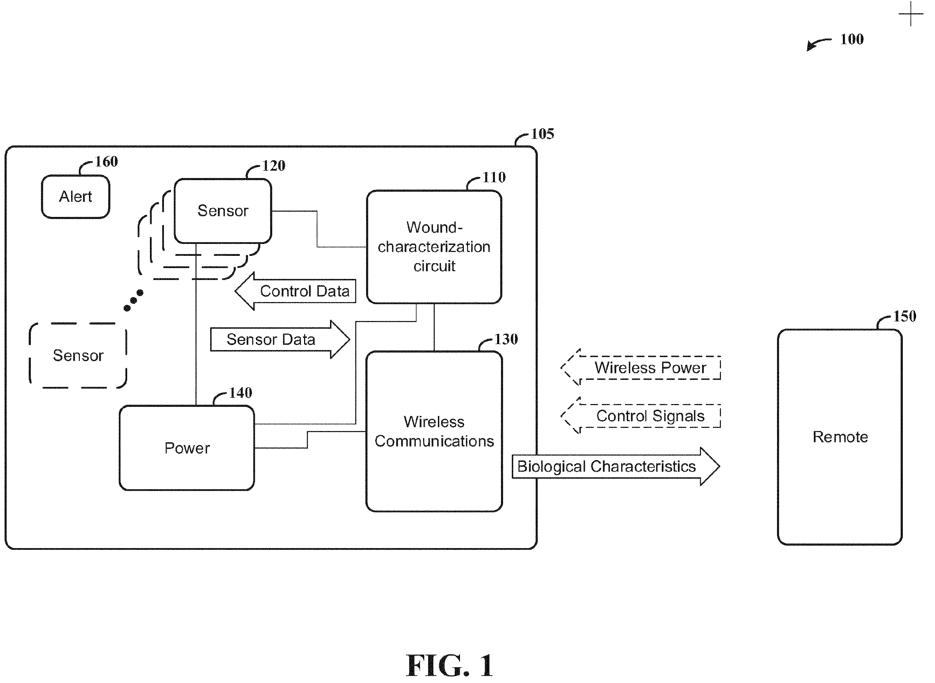

Turning now to the figures, FIG. 1 shows an apparatus 100 for wound monitoring, in accordance with an example embodiment. The apparatus 100 includes a component 105 including a wound-characterization circuit 110, one or more sensors (with sensor 120 labeled and additional sensors shown with dashed lines), wireless communication circuitry 130 and a power circuit 140. The wireless communication circuitry 130 carries out communications with a remote device 150, for conveying information relating to wound characteristics sensed by the one or more sensors 120. The wound-characterization circuit 110 communicates with the sensor(s) 120 for obtaining sensor data therefrom, and operates with the wireless communications circuit 130 to transmit data indicative of the sensor data, therein providing information that characterizes biological characteristics of a monitored wound.

The component 105 is implemented in a variety of manners to suit particular embodiments, and as such may be implemented in accordance with various embodiments as noted above. In some embodiments, one or more of the wound-characterization circuit 110, sensor 120 (or additional sensors), wireless communication circuit 130 and power circuit 140 are integrated in a common circuit. For instance, the wound-characterization circuit 110 may include one or more of the sensors 120, as an alternative to and/or in addition to other sensors 120 that are separate from the wound characterization circuit. In this context, the component 105 may be an integrated circuit chip, with the respective circuits being integrated thereupon. Various types of sensors as characterized herein can be implemented with one or more of the sensors (including sensor 120) as shown. In some implementations, the component 105 includes a wound dressing, upon which the noted circuits are affixed or in which the circuits are integrated.

The sensor data can be presented via the wireless communication circuit 130 using a variety of approaches. In some embodiments, raw sensor data is transmitted for processing at the remote device 150, or for relaying to another remote circuit (e.g., via a long-range network) where the data is processed. In other embodiments, the wound-characterization circuit 110 carries out some processing of raw sensor data, such as to configure the data for transmission or to provide some indication of a characteristic of a monitored wound. This approach may, for example, involve processing information from different types of sensors with an algorithm and/or using a comparison to threshold-type data that provides an indication of wound characteristics. For instance, where a combination of wound temperature and color provides an indication of infection, the wound-characterization circuit 110 may process temperature and color data relative to stored information to provide an indication of whether a wound is becoming infected.

In various embodiments, the wound-characterization circuit 110 controls the operation of one or more of the sensors 120. In some implementations, control data is sent to the sensors 120, such as for obtaining a reading at a particular interval. In other implementations, power is coupled to the sensor 120 for operating the sensor 120 in an active mode, at a selected time or interval. Different combinations of sensors 120 may be operated for certain types of wound assessment. Each sensor 120 returns sensor data to the wound-characterization circuit.

The power circuit 140 can be implemented as a wireless power circuit, as a battery or other power storage circuit, or a combination thereof. When implemented as a wireless power circuit, the power circuit 140 receives radio frequency (RF) or other wireless power as received via the wireless communications circuit 130 using a common or different antenna as is used for communicating with the remote device 150. Battery applications may be implemented for monitoring relatively serious wounds.

In some embodiments, the remote device 150 controls operation of the component 105 via wireless control signals. This approach may, for example, be carried out as discussed above to assess a wound via a particular sensor 120 or combination of sensors 120. This control may also set wound-monitoring intervals, or other characteristics regarding communications and alert conditions.

In some embodiments, the component 105 also includes an alert circuit 160. This circuit may, for example, include a light emitting diode(s) *(e.g.,* LEDs of different colors), a sound generation device or other circuit that can provide an indication of an alert condition. The alert circuit 160 is controlled by the wound-characterization circuit 110, based upon information gleaned from the one or more sensors 120. In some embodiments, the alert circuit 160 provides information about the wound, such as the degree to which a wound needs attention, or a type of issue discovered with the wound.

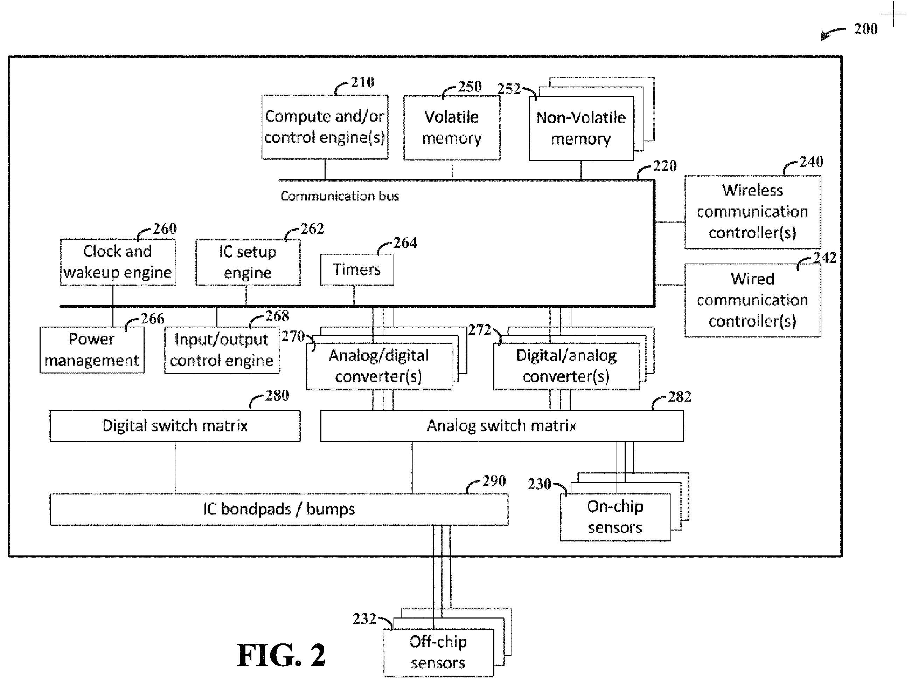

FIG. 2 shows another circuit apparatus 200 for wound monitoring, in accordance with another example embodiment. In various contexts, the apparatus 200 may be implemented for one or more components as shown in FIG. 1. The apparatus 200 includes compute and/or control engines 210, which communicate on a communication bus 220 and interact with a variety of circuits including sensors and communication circuits. By way of example, on-chip sensors 230 and off-chip sensors 232 are labeled accordingly, with two or more of each respective sensor implemented in various contexts. Similarly, wireless communication controller(s) 240 is shown, with a wired communication controller(s) 242, also shown by way of example.

A variety of other circuits are shown, some or all of which may be implemented for particular applications. These include volatile memory 250 and non-volatile memory 252, clock and wake-up engine 260, IC setup engine 262, timers 264, power management circuitry 266 and input/output control engine 268, all connected to communication bus 220. In addition, converters including at least one of analog/digital converter(s) 270 and digital/analog converter(s) 272 are connected to the communication bus 220. A digital switch matrix 280 and analog switch matrix 282 are also connected as shown, to the off-chip sensors 232 via IC bondpads/bumps 290, and to on-chip sensors 230 via the analog switch matrix.

Wound characteristic sensing is carried out with FIG. 2 in a variety of manners. For instance, one or more sensors may be implemented with oxygen (or other component) sensitive materials. Moisture level or conductivity may be sensed using external non-functionalized electrodes. Glucose, lactate or parameters that can be measured using an enzyme reaction, can be measured using electrodes functionalized with enzymes that give an electrochemical reaction with the substrates of interest. Bacterial content can be indirectly measured optically using color sensitive reaction, or indirectly using a pH measurement.

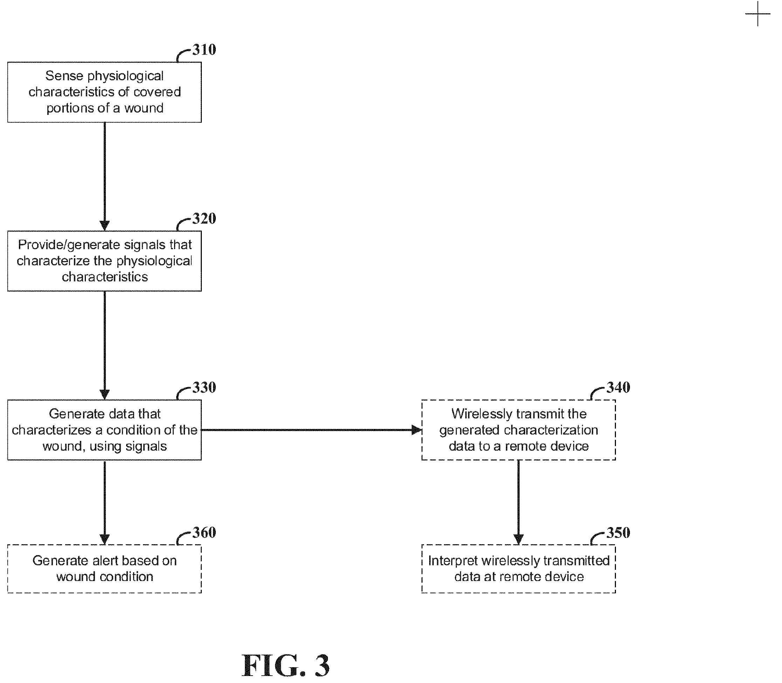

FIG. 3 shows a data flow diagram, in accordance with another example embodiment. At block 310, a sensor circuit integrated with a wound dressing that covers portions of a wound is used to sense physiological characteristics of the covered portions of the wound, and respective signals that characterize the physiological characteristics are provided at block 320. A wound-characterization circuit integrated with the sensor circuit and coupled to the wound dressing is used at block 330 to generate characterization data that characterizes a condition of the wound, based on physiological characteristics represented by the respective signals. These aspects are carried out while the wound dressing covers the portions of the wound, which facilitates monitoring of the wound without necessarily removing a wound dressing. At block 340, the characterization data is wirelessly transmitted to a remote device, such as to a handheld device that interprets or otherwise processes the data at block 350. In some implementations, an alert is generated at block 360, which may be carried out at one or both of the wound-characterization circuit and the remote device.

Various blocks, modules or other circuits may be implemented to carry out one or more of the operations and activities described herein and/or shown in the figures. In these contexts, a "block" (also sometimes "logic circuitry" or "module") is a circuit that carries out one or more of these or related operations/activities (e.g., sensing, controlling or alerting). For example, in certain of the above-discussed embodiments, one or more modules are discrete logic circuits or programmable logic circuits configured and arranged for implementing these operations/activities, as in the circuit modules shown in FIG. 1. In certain embodiments, such a programmable circuit is one or more computer circuits programmed to execute a set (or sets) of instructions (and/or configuration data). The instructions (and/or configuration data) can be in the form of firmware or software stored in and accessible from a memory (circuit). As an example, first and second modules include a combination of a CPU hardware-based circuit and a set of instructions in the form of firmware, where the first module includes a first CPU hardware circuit with one set of instructions and the second module includes a second CPU hardware circuit with another set of instructions.

Certain embodiments are directed to a computer program product (e.g., nonvolatile memory device), which includes a machine or computer-readable medium having stored thereon instructions which may be executed by a computer (or other electronic device) to perform these operations/activities.
Embodiments may include features as recited in the following numbered clauses.
1. An apparatus comprising: a sensor circuit; a wound-characterization circuit configured and arranged to respond to signals provided from the sensor circuit by generating characterization data that characterizes a condition of a wound based on physiological characteristics represented by the respective signals; a wireless communication circuit configured and arranged with the wound-characterization circuit to wirelessly transmit the characterization data; a power circuit configured and arranged to provide power to the wound-characterization circuit and to the wireless communication circuit; and an integration circuit, including the wound-characterization circuit and the sensor circuit, configured and arranged for coupling to a wound dressing and for sensing different physiological characteristics of the wound while the integration circuit and the wound dressing cover at least a portion of the wound.
2. The apparatus of clause 1, wherein the sensor circuit includes a plurality of sensors, each sensor being configured and arranged to sense a physiological characteristic that is different than a physiological characteristic sensed by at least another one of the sensors.
3. The apparatus of clause 1 or 2, wherein the integration circuit includes a substrate configured and arranged to adhere to the wound dressing and to affix the sensor circuit, wound-characterization circuit, wireless communication circuit, and power circuit relative to one another.
4. The apparatus of any of clauses 1 to 3, wherein the wound-characterization circuit is configured and arranged to generate the characterization data by converting at least two of the signals into digital data and executing an algorithm using the digital data as inputs, based upon the type of physiological characteristics represented by the respective signals, and generating the characterization data therefrom that characterizes a condition of the wound based on a combination of different physiological characteristics represented by respective ones of the at least two signals.
5. The apparatus of any of clauses 1 to 4, wherein the sensor circuit includes a plurality of sensors, each sensor being configured and arranged to characterize a physiological characteristic that is different than a physiological characteristic sensed by at least another one of the sensors.
6. The apparatus of clause 5, further including a memory circuit configured and arranged to store data representing wound condition-sensing protocols specific to different wound types, each protocol specifying a combination of sensors to be used, and wherein the wound-characterization circuit is a logic circuit configured and arranged to monitor different types of wounds by:retrieving one of the sensing protocols specific to a particular type of wound, based upon identification data specifying the type of wound to be sensed, and selectively operating different combinations of the plurality of sensors, by retrieving and executing one of the protocols based on the identification data.
7. The apparatus of clause 6, wherein the logic circuit is configured and arranged to process data from the sensors to determine a characteristic of the wound, and to generate and transmit an alert via the wireless communication circuit in response to the determined characteristic of the wound indicating a medical condition defined in the memory as being adverse.
8. The apparatus of any of clauses 1 to 7, wherein the wound-characterization circuit is configured and arranged to process data from the sensor circuit to determine a characteristic of the wound, and to generate and transmit an alert via the wireless communication circuit in response to the determined characteristic of the wound indicating a medical condition defined as being adverse.
9. The apparatus of any of clauses 1 to 8, wherein the wireless communication circuit is a near-field communication (NFC) circuit configured and arranged with the power circuit to receive wireless power and to use the wireless power to power to the wound-characterization circuit and to the wireless communication circuit, and the wound-characterization circuit is configured and arranged to operate the sensor circuit to obtain the physiological characteristics and to transmit the characterization data via the NFC circuit.
10. The apparatus of clause 9, further including a portable device having an NFC circuit and being configured and arranged to transmit the wireless power to the wireless communication circuit via the NFC circuits, and remotely operate the sensor circuit and the wound-characterization circuit for detecting the physiological characteristics by communicating instructions to the wound-characterization circuit via the NFC circuits.
11. The apparatus of clause 10, wherein the sensor circuit includes a plurality of sensors, each sensor being configured and arranged to characterize a physiological characteristic that is different than a physiological characteristic sensed by at least another one of the sensors, and wherein the portable device is configured and arranged to remotely operate the sensor circuit, the NFC circuit and the wound-characterization circuit by controlling the wound-characterization circuit to: operate at least a first one of the sensors for detecting a biochemical characteristic of the wound, and transmit the characterization data to the portable device, and in response to the characterization data, operate at least a second one of the sensors for detecting a physiological characteristic of the wound, the at least a second one of the sensors being different than the at least a first one of the sensors, and transmit data including the detected biochemical characteristic of the wound to the portable device.
12. The apparatus of any of clauses 1 to 11, wherein the sensor circuit is configured and arranged to sense at least two physiological characteristics selected from the group of: an electrical characteristic, a chemical characteristic, a mechanical characteristic, a temperature characteristic and an optical characteristic.
13. The apparatus of any of clauses 1 to 12, further including the wound dressing, the wound dressing being configured and arranged with the integration circuit to support and adhere the sensor circuit, wound-characterization circuit, wireless communication circuit and power circuit in contact with a patient exhibiting the wound, thereby providing assessment of a portion of the wound covered by the wound dressing while the wound dressing is covering the portion of the wound.
14. The apparatus of any of clauses 1 to 13, wherein the sensor circuit includes at least two sensors spaced apart from one another and configured and arranged with the integration circuit to characterize different regions of the wound that are spaced apart from one another.
15. An apparatus comprising: a first circuit configured and arranged for coupling to a wound dressing, the first circuit comprising: a plurality of sensors configured and arranged to sense different physiological characteristics of a wound while the wound dressing covers at least a portion of the wound from which the physiological characteristics are sensed; a wound-characterization circuit configured and arranged to respond to signals provided from the sensors by generating characterization data that characterizes a condition of the wound based on physiological characteristics represented by the respective signals; and a wireless communication circuit configured and arranged with the wound-characterization circuit to wirelessly transmit the characterization data; and a second circuit configured and arranged to wirelessly communicate with the first circuit to receive the characterization data via the wireless communication circuit; and provide an indication of a condition of the wound based on the characterization data.
16. The apparatus of clause 15, wherein the second circuit is configured and arranged to power the first circuit by transmitting wireless power to the first circuit, the first circuit being configured and arranged to harvest and use the wireless power to operate the sensors, wound-characterization circuit and wireless communication circuit for characterizing the portion of the wound covered by the wound dressing.
17. The apparatus of clause 15 or 16, wherein the second circuit is configured and arranged with the first circuit to remotely control the first circuit by transmitting operational instructions for controlling selected ones of the sensors for sensing specific physiological characteristics, and the first circuit is configured and arranged to respond to the operational instructions by operating the sensors to sense the specific physiological characteristics and by generating characterization data, via the wound-characterization circuit, that characterizes a condition of the wound based on the operational instructions.
18. A method comprising: using a sensor circuit integrated with a wound dressing that covers portions of a wound, sensing physiological characteristics of the covered portions of the wound and providing respective signals that characterize the physiological characteristics; using a wound-characterization circuit integrated with the sensor circuit and coupled to the wound dressing, generating characterization data that characterizes a condition of the wound based on physiological characteristics represented by the respective signals, while the wound dressing covers the portions of the wound; and wirelessly transmitting the characterization data.
19. The method of clause 18, wherein sensing the physiological characteristics of the covered portions of the wound includes sensing at least two different physiological characteristics of the wound, and generating the characterization data includes generating characterization data based on a combination of the at least two different physiological characteristics.
20. The method of clause 19, further including: receiving and using the wirelessly transmitted characterization data to identify a condition of the wound, and in response to the identified condition of the wound indicating an adverse condition, generating an alert that alerts a user of the adverse condition while the wound dressing covers the portion of the wound.

As may be implemented with one or more embodiments herein, aspects of the disclosure are directed to an apparatus having one or more sensor circuits and a wound-characterization circuit that generates data that characterizes a condition of the wound based on physiological characteristics detected by the sensors. A wireless communication circuit wirelessly transmits the characterization data, which can be carried out while the wound is covered. In various implementations, an integration circuit (e.g., connecting circuitry and/or a chip) includes the wound-characterization circuit and the sensor circuit, and further couples to a wound dressing for sensing the different physiological characteristics of the wound while the wound dressing covers a portion of the wound from which the different physiological characteristics are sensed.

Based upon the above discussion and illustrations, those skilled in the art will readily recognize that various modifications and changes may be made to the various embodiments without strictly following the exemplary embodiments and applications illustrated and described herein. For example, a variety of different types of sensors can be used to sense various different characteristics. In addition, power may be provided in a variety of manners, and communications may be effected using different types of wireless communications. In some embodiments, wired communications are used. Such modifications do not depart from the true spirit and scope of various aspects of the invention, including aspects set forth in the claims.

## Claims

1. An apparatus comprising:
a sensor circuit;
a wound-characterization circuit configured and arranged to respond to signals provided from the sensor circuit by generating characterization data that characterizes a condition of a wound based on physiological characteristics represented by the respective signals;
a wireless communication circuit configured and arranged with the wound-characterization circuit to wirelessly transmit the characterization data;
a power circuit configured and arranged to provide power to the wound-characterization circuit and to the wireless communication circuit; and
an integration circuit, including the wound-characterization circuit and the sensor circuit, configured and arranged for coupling to a wound dressing and for sensing different physiological characteristics of the wound while the integration circuit and the wound dressing cover at least a portion of the wound.

2. The apparatus of claim 1, wherein the sensor circuit includes a plurality of sensors, each sensor being configured and arranged to sense a physiological characteristic that is different than a physiological characteristic sensed by at least another one of the sensors.

3. The apparatus of any preceding claim, wherein the integration circuit includes a substrate configured and arranged to adhere to the wound dressing and to affix the sensor circuit, wound-characterization circuit, wireless communication circuit, and power circuit relative to one another.

4. The apparatus of any preceding claim, wherein the wound-characterization circuit is configured and arranged to generate the characterization data by converting at least two of the signals into digital data and executing an algorithm using the digital data as inputs, based upon the type of physiological characteristics represented by the respective signals, and generating the characterization data therefrom that characterizes a condition of the wound based on a combination of different physiological characteristics represented by respective ones of the at least two signals.

5. The apparatus of any preceding claim, wherein the sensor circuit includes a plurality of sensors, each sensor being configured and arranged to characterize a physiological characteristic that is different than a physiological characteristic sensed by at least another one of the sensors.

6. The apparatus of claim 5,
further including a memory circuit configured and arranged to store data representing wound condition-sensing protocols specific to different wound types, each protocol specifying a combination of sensors to be used, and
wherein the wound-characterization circuit is a logic circuit configured and arranged to monitor different types of wounds by:
retrieving one of the sensing protocols specific to a particular type of wound, based upon identification data specifying the type of wound to be sensed, and
selectively operating different combinations of the plurality of sensors, by retrieving and executing one of the protocols based on the identification data.

7. The apparatus of claim 6, wherein the logic circuit is configured and arranged to process data from the sensors to determine a characteristic of the wound, and to generate and transmit an alert via the wireless communication circuit in response to the determined characteristic of the wound indicating a medical condition defined in the memory as being adverse.

8. The apparatus of any preceding claim, wherein the wound-characterization circuit is configured and arranged to process data from the sensor circuit to determine a characteristic of the wound, and to generate and transmit an alert via the wireless communication circuit in response to the determined characteristic of the wound indicating a medical condition defined as being adverse.

9. The apparatus of any preceding claim,
wherein the wireless communication circuit is a near-field communication (NFC) circuit configured and arranged with the power circuit to receive wireless power and to use the wireless power to power to the wound-characterization circuit and to the wireless communication circuit, and
the wound-characterization circuit is configured and arranged to operate the sensor circuit to obtain the physiological characteristics and to transmit the characterization data via the NFC circuit.

10. The apparatus of claim 9, further including a portable device having an NFC circuit and being configured and arranged to
transmit the wireless power to the wireless communication circuit via the NFC circuits, and
remotely operate the sensor circuit and the wound-characterization circuit for detecting the physiological characteristics by communicating instructions to the wound-characterization circuit via the NFC circuits.

11. The apparatus of claim 10, wherein the sensor circuit includes a plurality of sensors, each sensor being configured and arranged to characterize a physiological characteristic that is different than a physiological characteristic sensed by at least another one of the sensors, and wherein the portable device is configured and arranged to remotely operate the sensor circuit, the NFC circuit and the wound-characterization circuit by controlling the wound-characterization circuit to:
operate at least a first one of the sensors for detecting a biochemical characteristic of the wound, and
transmit the characterization data to the portable device, and
in response to the characterization data,
operate at least a second one of the sensors for detecting a physiological characteristic of the wound, the at least a second one of the sensors being different than the at least a first one of the sensors, and
transmit data including the detected biochemical characteristic of the wound to the portable device.

12. The apparatus of any preceding claim, wherein the sensor circuit is configured and arranged to sense at least two physiological characteristics selected from the group of: an electrical characteristic, a chemical characteristic, a mechanical characteristic, a temperature characteristic and an optical characteristic.

13. The apparatus of any preceding claim, further including the wound dressing, the wound dressing being configured and arranged with the integration circuit to support and adhere the sensor circuit, wound-characterization circuit, wireless communication circuit and power circuit in contact with a patient exhibiting the wound, thereby providing assessment of a portion of the wound covered by the wound dressing while the wound dressing is covering the portion of the wound.

14. The apparatus of any preceding claim, wherein the sensor circuit includes at least two sensors spaced apart from one another and configured and arranged with the integration circuit to characterize different regions of the wound that are spaced apart from one another.

15. A method comprising:
using a sensor circuit integrated with a wound dressing that covers portions of a wound, sensing physiological characteristics of the covered portions of the wound and providing respective signals that characterize the physiological characteristics;
using a wound-characterization circuit integrated with the sensor circuit and coupled to the wound dressing, generating characterization data that characterizes a condition of the wound based on physiological characteristics represented by the respective signals, while the wound dressing covers the portions of the wound; and
wirelessly transmitting the characterization data.
